# EUROPÄISCHE PATENTANMELDUNG

(11) **EP 4 841 192 A1**
(43) Veröffentlichungstag der Anmeldung: **18.11.2026**
(21) Anmeldenummer: 26173439.6
(22) Anmeldetag: 20.04.2026
(51) Int. Cl.: F25D 23/12, A61L 2/10

(54) **VERFAHREN ZUR UNTERSTÜTZUNG EINES NUTZERS BEI EINER REINIGUNG EINES HAUSHALTGERÄTS MIT UV-LICHTQUELLE UND BILDERFASSUNGSEINRICHTUNG, VORRICHTUNG ZUR UNTERSTÜTZUNG EINES NUTZERS BEI EINER REINIGUNG EINES HAUSHALTGERÄTS UND HAUSHALTGERÄT**

(30) Priorität: 12.05.2025 LU 601543
(71) Anmelder: Miele & Cie. KG, 33332 Gütersloh (DE)
(72) Erfinder: Westergerling, Mario, 33334 Gütersloh (DE); Schmelter, Stefan, 33330 Gütersloh (DE); Franzmeier, Jens, 32052 Herford (DE)

(57) **Zusammenfassung**

Die Erfindung betrifft ein Verfahren zur Unterstützung eines Nutzers bei einer Reinigung eines Haushaltgeräts (100), das eine UV-Lichtquelle (205) zum Beleuchten eines Innenraums des Haushaltgeräts (100) und eine Bilderfassungseinrichtung (210) zum Erfassen eines Abbilds (320, 325) des Innenraums aufweist, mit einem Schritt des Aktivierens eines Reinigungsmodus zum Ermöglichen eines Reinigungsvorgangs des Innenraums ansprechend auf ein über eine Benutzerschnittstelle (311) eingelesenes Aktivierungssignal (315), einem Schritt des Einschaltens der UV-Lichtquelle (205) nach einem Abschluss des Reinigungsvorgangs, einem Schritt des Erfassens eines zweiten Abbilds (320) des Innenraums unter Verwendung der Bilderfassungseinrichtung (210) während die UV-Lichtquelle (205) eingeschaltet ist, einem Schritt des Ausschaltens der UV-Lichtquelle (205) und einem Schritt des Bereitstellens eines ersten Abbilds (325) und des zweiten Abbilds (320) für einen Vergleich zum Feststellen eines Reinigungsergebnisses des Reinigungsvorgangs.

## Beschreibung

Die Erfindung betrifft ein Verfahren zur Unterstützung eines Nutzers bei einer Reinigung eines Haushaltgeräts mit UV-Lichtquelle und Bilderfassungseinrichtung, eine Vorrichtung zur Unterstützung eines Nutzers bei einer Reinigung eines Haushaltgeräts und ein Haushaltgerät.

Die Reinigung von Haushaltgeräten, besonders von Kühlgeräten, ist aufwändig und häufig negativ konnotiert. Verschmutzungen im Haushaltgerät sind nicht immer für das menschliche Auge sichtbar. Weiterhin hat kaum jemand vor Augen, wann beispielsweise der Kühlschrank das letzte Mal gereinigt wurde.

Der hier vorgestellte Ansatz stellt sich die Aufgabe, ein verbessertes Verfahren zur Unterstützung eines Nutzers bei einer Reinigung eines Haushaltgeräts, eine verbesserte Vorrichtung zur Unterstützung eines Nutzers bei einer Reinigung eines Haushaltgeräts und ein verbessertes Haushaltgerät zu schaffen.

Erfindungsgemäß wird diese Aufgabe durch ein Verfahren zur Unterstützung eines Nutzers bei einer Reinigung eines Haushaltgeräts, eine Vorrichtung zur Unterstützung eines Nutzers bei einer Reinigung eines Haushaltgeräts und ein Haushaltgerät mit den Merkmalen der Hauptansprüche gelöst. Vorteilhafte Ausgestaltungen und Weiterbildungen der Erfindung ergeben sich aus den nachfolgenden Unteransprüchen.

Die mit der Erfindung erreichbaren Vorteile bestehen darin, dem Nutzer Verschmutzungen aufzeigen zu können, welche dieser nicht mit bloßem Auge erkennen könnte. Dadurch kann der Nutzer das Ergebnis einer Reinigung besser beurteilen. Zudem wird dem Nutzer die Möglichkeit gegeben, das Ergebnis seiner Reinigung mit einem gewünschten Sauberkeitszustands des Haushaltgeräts zu vergleichen und so auch noch einmal die Reinigung gezielter und somit effizienter durchführen zu können.

Vorteilhafterweise kann dem Nutzer eine Assistenzfunktion zur Hand gegeben werden, um die Hygiene im eigenen Haushaltgerät bewerten zu können. Weiterhin soll der Assistent dazu dienen, den Reinigungsprozess des Haushaltgeräts zu unterstützen und zu optimieren.

Ein Verfahren zur Unterstützung eines Nutzers bei einer Reinigung eines Haushaltgeräts, das eine UV-Lichtquelle zum Beleuchten eines Innenraums des Haushaltgeräts und eine Bilderfassungseinrichtung zum Erfassen eines Abbilds des Innenraums aufweist, umfasst die folgenden Schritte:
Aktivieren eines Reinigungsmodus zum Ermöglichen eines Reinigungsvorgang des Innenraums ansprechend auf ein über eine Benutzerschnittstelle eingelesenes Aktivierungssignal;
Einschalten der UV-Lichtquelle nach einem Abschluss des Reinigungsvorgangs;
Erfassen eines zweiten Abbilds des Innenraums unter Verwendung der Bilderfassungseinrichtung, während die UV-Lichtquelle eingeschaltet ist;
Ausschalten der UV-Lichtquelle; und
Bereitstellen eines ersten Abbilds und des zweiten Abbilds für einen Vergleich zum Feststellen eines Reinigungsergebnisses des Reinigungsvorgangs.

Bei dem Haushaltgerät kann es sich beispielsweise um ein Kühlgerät wie einen Kühlschrank oder einen Gefrierschrank handeln. Durch die Lagerung von verderblichen Lebensmitteln können hier schnell Verschmutzungen entstehen und häufige Reinigungen notwendig machen, weshalb sich hier ein Verfahren, wie es hier beschrieben wird, anbieten kann.

Bei der Bilderfassungseinrichtung kann es sich beispielsweise um eine Kamera handeln, welche den Innenraum des Haushaltgeräts abbilden kann. Die Bilderfassungseinrichtung kann dabei beispielsweise an einer Tür des Haushaltgeräts angeordnet sein, um den gesamten Innenraum des Haushaltgeräts erfassen zu können.

Unter einem Reinigungsmodus, synonym als Cleaning Mode bezeichnet, kann ein vorprogrammierter Modus verstanden werden, welcher optimale Bedingungen für eine Reinigung des Haushaltgeräts schaffen kann. Bei einer Ausführungsform des Haushaltgeräts als Kühlschrank beispielsweise kann im Reinigungsmodus der Kompressor ausgeschaltet sein, aber eine Beleuchtung des Innenraums eingeschaltet bleiben. Der Reinigungsmodus kann beispielsweise direkt durch den Nutzer einschaltbar sein. Der Modus kann dabei so konfiguriert sein, dass er sich automatisch nach einer vordefinierten Zeit von alleine wieder abschaltet, sodass das Haushaltgerät dann wieder ordnungsgemäß läuft.

Unter einer Benutzerschnittstelle kann eine Einheit verstanden werden, über welche der Nutzer in der Lage sein kann, mit einer Steuerung des Haushaltgeräts zu kommunizieren, beispielsweise durch eine Eingabe an einem Tastenfeld oder Touchscreen. Die Benutzerschnittstelle kann jedoch auch beispielsweise als ein mobiles Endgerät, wie ein Mobiltelefon, ausgeformt sein, welches beispielsweise über Funk, Bluetooth, oder eine andere drahtlose Verbindung mit dem Haushaltgerät gekoppelt sein kann.

Das zweite Abbild kann ein Abbild des Innenraums des Haushaltgeräts nach der aktuell durchzuführenden Reinigung sein. Das erste Abbild kann wiederum ein Abbild des Innenraums des Haushaltgeräts vor der aktuell durchzuführenden Reinigung sein. Gemäß einer Ausführungsform kann das erste Abbild beispielsweise ein bereits vorhandenes Abbild des Innenraums des Haushaltgeräts in einem optimal gereinigten Zustand sein.

Gemäß dieser Ausführungsform kann hier beispielsweise durch einen Vergleich der beiden Abbilder festgestellt werden, ob das Haushaltgerät nach der Reinigung, wie es im zweiten Abbild abgebildet ist, einem Idealzustand entspricht, welcher durch das erste Abbild abgebildet sein kann. Durch einen solchen Vergleich zwischen einem Ist-Zustand in Form des zweiten Abbilds und einem Soll-Zustand in Form des ersten Abbilds kann sich leicht beurteilen lassen, ob die Reinigung zufriedenstellend war. Wenn das erste Abbild den Zustand vor der Reinigung abbildet, kann durch den Vergleich ein Reinigungserfolg überprüft werden.

Im Zusammenhang mit einem Kühlgerät, wie einem Kühlschrank, ist der hier beschriebene Ansatz vorteilhaft, da dauerhafte und starke Verunreinigungen Krankheiten verursachen können. Allgemein kann eine schlechte Hygiene im Umgang mit Lebensmitteln gesundheitliche Folgen mit sich ziehen.

Im Lebensmittelbereich eignet sich UV-Licht, um Verunreinigungen zu erkennen. Automatisierte Systeme können dabei Verunreinigungen wie Schimmel oder Bakterien auf Lebensmitteln und Verpackungen identifizieren und so die Lebensmittelsicherheit erhöhen. Vorteilhafterweise hat der Nutzer dabei eine Entscheidungsgewalt, inwieweit er das gegebene System nutzen möchte. Im Gegensatz zu einem hoch automatisierten Prozess kann der hier vorgeschlagene Ansatz vom Verhalten des Nutzers abhängig sein. Der Nutzer kann also aufgeklärt werden, ohne dabei bevormundet zu werden.

Im Schritt des Bereitstellens kann das erste Abbild als ein vorbestimmtes Referenzbild aus einer Speichereinrichtung ausgelesen werden. Das vorbestimmte Referenzbild kann beispielsweise bei einer ersten Inbetriebnahme des Haushaltgeräts erfasst werden, oder aber auch bereits von Herstellerseite bereitgestellt sein. Das vorbestimmte Referenzbild kann somit einen Zustand des Innenraums des Haushaltgeräts vor jeglicher Verschmutzung abbilden. Ein solches vorbestimmtes Referenzbild kann also eine einfache Möglichkeit darstellen, einen Idealzustand des Haushaltgeräts vorzugeben und den aktuellen Zustand des Innenraums Haushaltgeräts anhand eines Bildabgleichs beurteilen zu können. Die Speichereinrichtung kann sich dabei um Haushaltgerät befinden oder beispielsweise in einer Cloud oder einem mobilen Gerät.

Das Verfahren kann einen Schritt des Einschaltens der UV-Lichtquelle ansprechend auf das Aktivieren des Reinigungsmodus, einen Schritt des Erfassens des ersten Abbilds des Innenraums unter Verwendung der Bilderfassungseinrichtung während die UV-Lichtquelle eingeschaltet ist, und einen Schritt des Ausschaltens der UV-Lichtquelle umfassen, um den Reinigungsvorgang zu ermöglichen. Gemäß einer Ausführungsform kann das erste Abbild hier ein Abbild des Innenraums des Haushaltgeräts unmittelbar vor dem Reinigen und somit in einem verschmutzten Zustand darstellen. Hierdurch kann eine effektive Möglichkeit geschaffen werden, das Ergebnis der soeben durchgeführten Reinigung festzustellen. Außerdem kann so möglicherweise auch vor einem endgültigen Abschließen der Reinigung der Reinigungsfortschritt durch den direkten Vergleich mit dem ersten Abbild beurteilt werden.

Im Schritt des Bereitstellens kann der Vergleich des ersten Abbilds und des zweiten Abbilds durchgeführt werden und abhängig von einem Ergebnis des Vergleichs ein Ergebnissignal bereitgestellt werden, das anzeigt, ob der Reinigungsvorgang zu einem zufriedenstellenden Reinigungsergebnis oder einem nicht-zufriedenstellenden Reinigungsergebnis geführt hat.

Gemäß einer Ausführungsform kann der Vergleich beispielsweise mittels einer KI durchgeführt werden, welche ausgebildet sein kann, das Reinigungsergebnis anhand dieses Vergleichs zu beurteilen.

Das Ergebnissignal kann dabei dieses Reinigungsergebnis repräsentieren. Ein zufriedenstellendes Reinigungsergebnis kann beispielsweise dann signalisiert werden, wenn festgestellt wird, dass das erste Abbild und das zweite Abbild weitestgehend übereinstimmen, wenn das erste Abbild hier ein vorbestimmtes Referenzbild des Haushaltgeräts im gereinigten Zustand darstellt. Analog dazu kann ein nichtzufriedenstellendes Reinigungsergebnis signalisiert werden, wenn das erste Abbild und das zweite Abbild nicht ausreichend übereinstimmen.

Gemäß einer Ausführungsform, bei welchem das erste Abbild unmittelbar vor der Reinigung erfasst wird und das Haushaltgerät somit im schmutzigen Zustand abbildet kann wiederum ein zufriedenstellendes Reinigungsergebnis signalisiert werden, wenn sich das erste und das zweite Abbild möglichst unterscheiden und ein nichtzufriedenstellendes Reinigungsergebnis signalisiert werden, wenn sich die Abbilder nicht ausreichend unterscheiden.

Ein solcher Vergleich zweier Abbilder des Haushaltgeräts vor und nach der Reinigung kann somit eine effiziente Möglichkeit bieten, um das Ergebnis der Reinigung zu beurteilen. Durch einen automatischen Vergleich, beispielsweise mittels KI, können außerdem auch übrige Verschmutzungen erkannt werden, welche ein Nutzer gegebenenfalls mit bloßem Auge nicht erkennen könnte.

Das Verfahren kann einen Schritt des Ausgebens eines Hinweises über die Benutzerschnittstelle umfassen, wenn das Ergebnissignal das nichtzufriedenstellende Reinigungsergebnis anzeigt. Der Hinweise kann beispielsweise als visuelles oder auch als akustisches Signal an der Benutzerschnittstelle ausgegeben werden. So kann dem Nutzer leicht mitgeteilt werden, dass eine Reinigung nicht zufriedenstellend war und sie gegebenenfalls wiederholt werden sollte.

Im Schritt des Bereitstellens können das erste Abbild und das zweite Abbild an die Benutzerschnittstelle zur Anzeige für den Nutzer bereitgestellt werden. Über die Benutzerschnittstelle kann ein Ergebnissignal bereitgestellt werden, das anzeigt, ob der Reinigungsvorgang zu einem zufriedenstellenden Reinigungsergebnis oder einem nichtzufriedenstellenden Reinigungsergebnis geführt hat.

Eine Anzeige der beiden Abbilder kann gemäß einer Ausführungsform beispielsweise gegebenenfalls über ein Display des Haushaltgeräts selbst oder auch über ein mobiles Endgerät wie ein Mobiltelefon ausgegeben werden.

Das Ergebnissignal kann anzeigen, ob das Reinigungsergebnis aus Sicht des Nutzers zufriedenstellend ist. Zum Bereitstellen des Ergebnissignals kann beispielsweise eine Eingabe des Nutzers an der Benutzerschnittstelle möglich sein. Je nach Ausführungsform der Benutzerschnittstelle kann hier eine Tasten- oder Touchscreen-Eingabe vorgenommen werden. Eine weitere Möglichkeit wäre auch eine Ausführungsform, bei der eine Spracheingabe an der Benutzerschnittstelle möglich ist. Somit kann das Ergebnissignal ansprechend auf eine Eingabe des Nutzers eingelesen werden.

Der Vorteil des Bereitstellens des Ergebnissignals über die Benutzerschnittstelle kann sein, dass der Nutzer hier selbst entscheiden kann, ob er mit dem Reinigungsergebnis zufrieden ist oder nicht. So kann verhindert werden, dass der Nutzer durch die Beurteilung durch beispielsweise eine KI zu sehr bevormundet wird.

Das Verfahren kann einen Schritt des Abspeicherns des zweiten Abbilds als ein vorbestimmtes Referenzbild in eine Speichereinrichtung umfassen, wenn das Ergebnissignal das zufriedenstellende Reinigungsergebnis anzeigt. Somit kann das zweite Abbild verwendet werden, um das vorhandene erste Abbild durch ein aktuelles erstes Abbild zu ersetzen.

Das vorbestimmte Referenzbild kann gemäß einer Ausführungsform des hier beschriebenen Ansatzes dann den idealen sauberen Zustand des Haushaltgeräts abbilden und ein gegebenenfalls vorher vorhandenes Referenzbild ersetzen. Falls es jedoch kein vorher vorhandenes Referenzbild gibt, besteht hiermit die Möglichkeit, ein solches einzuspeichern und für zukünftige Reinigungen zu nutzen.

Gemäß einer Ausführungsform kann es auch möglich sein, dass ein Nutzer selbst bestimmen kann, ob er das zweite Abbild als ein vorbestimmtes Referenzbild speichern möchte.

Wenn die Reinigung als zufriedenstellend beurteilt wurde, kann davon ausgegangen werden, dass das zweite Abbild in diesem Fall das Haushaltgerät in einem Zustand abbildet, welcher möglichst auch bei jeder zukünftigen Reinigung erreicht werden sollte, weshalb es sinnvoll sein kann, dieses Abbild als zukünftiges Referenzbild zu verwenden.

Gemäß einer Ausführungsform, bei welcher der Nutzer selbst beurteilt, ob das Reinigungsergebnis zufriedenstellend ist, kann es auch insofern von Vorteil sein, das zweite Abbild als Referenzbild abzuspeichern, da dies genau den Zustand abbilden kann, den sich der Nutzer individuell als Idealzustand wünscht und welchen er bei jeder Reinigung erzielen möchte.

Das Verfahren kann einen Schritt des Vermerkens eines Reinigungsdatums und einen Schritt des Deaktivierens des Reinigungsmodus umfassen, wenn das Ergebnissignal das zufriedenstellende Reinigungsergebnis anzeigt.

Unter dem Reinigungsdatum kann das Datum speziell der im Verfahren durchgeführten Reinigung verstanden werden. Durch das Vermerken des Reinigungsdatums kann somit für die Zukunft festgehalten werden, wann das Haushaltgerät zuletzt gereinigt wurde. So kann der Nutzer beispielsweise immer einsehen, wie lange die letzte Reinigung zurückliegt. Außerdem kann diese Information für weitere, nachfolgend näher beschriebene, Schritte des Verfahrens verwendet werden.

Ein Deaktivieren des Reinigungsmodus ansprechend auf das zufriedenstellende Reinigungsergebnis kann auch sinnvoll sein, da naheliegend sein kann, dass bei einem zufriedenstellenden Reinigungsergebnis der Reinigungsvorgang beendet wird. So kann das Haushaltgerät wieder in den normalen Betriebsmodus wechseln.

Nach Ablauf einer vorbestimmten Zeitspanne nach dem Reinigungsdatum können ein Schritt des Einschaltens der UV-Lichtquelle, ein Schritt des Erfassens eines aktuellen zweiten Abbilds, ein Schritt des Ausschaltens der UV-Lichtquelle und ein Schritt des Bereitstellens eines ersten Abbilds und des aktuellen zweiten Abbilds für einen Vergleich zum Feststellen eines aktuellen Sauberkeitszustands des Innenraums ausgeführt werden, wobei im Schritt des Bereitstellens der Vergleich des ersten Abbilds und des aktuellen zweiten Abbilds durchgeführt werden kann und abhängig von einem Ergebnis des Vergleichs ein Zustandssignal bereitgestellt werden kann, das eine Sauberkeit oder Verschmutzung des Innenraums anzeigen kann.

Die vorbestimmte Zeitspanne kann beispielsweise eine Zeitspanne sein, nach der es sinnvoll wäre, eine weitere Reinigung vorzunehmen. Dabei kann es sich beispielsweise um zwei Wochen handeln. Gemäß einer Ausführungsform des hier vorgeschlagenen Ansatzes kann der Nutzer möglicherweise auch selbst die Zeitspanne festlegen.

Das aktuelle zweite Abbild kann also ein Abbild des Innenraums des Haushaltgeräts zum Zeitpunkt nach dem Ablauf der vorbestimmten Zeitspanne darstellen. Hieran kann also der aktuelle Sauberkeitszustand des Haushaltgeräts bestimmt werden.

Das erste Abbild wiederum kann ein Abbild des Haushaltgeräts im sauberen Zustand sein. Dabei kann es sich gemäß einer Ausführungsform beispielsweise um das vorhandene Referenzbild handeln, oder aber auch um das als vorbestimmtes Referenzbild gespeicherte zweite Abbild aus der vorangegangenen Reinigung.

Anhand dieser beiden Abbilder kann sich nun der aktuelle Sauberkeitszustand des Haushaltgeräts beurteilen lassen. Das erste Abbild kann hier den Soll-Zustand darstellen. Durch einen Vergleich mit dem aktuellen zweiten Abbild, welches hier den Ist-Zustand des Haushaltgeräts darstellen kann, kann ein Verschmutzungsgrad und somit der Sauberkeitszustand festgestellt werden.

Dieser Sauberkeitszustand kann dann durch das Zustandssignal repräsentiert werden, wobei das Zustandssignal entweder eine Sauberkeit oder eine Verschmutzung anzeigen kann. Stimmen das erste und aktuelle zweite Abbild weitestgehend überein, kann das bedeuten, dass das Haushaltgerät noch hinreichend sauber ist und keine Reinigung nötig ist. Unterscheiden sich die beiden Abbilder jedoch stark, so kann von einer Verschmutzung ausgegangen werden, welche eine Reinigung notwendig machen kann.

Durch einen solchen Vergleich des Ist-Zustands und des Soll-Zustands nach der vorbestimmten Zeitspanne kann effizient festgestellt werden, ob eine Reinigung notwendig ist, was auch nach der Zeitspanne nicht immer der Fall sein muss. Somit kann eine Zeitverschwendung für den Nutzer durch eine unnötige Reinigung vermieden werden.

Das Verfahren kann einen Schritt des Ausgebens eines Anfragesignals zum Anfragen nach einem Reinigungsvorgangs über die Benutzerschnittstelle umfassen, wenn das Zustandssignal die Verschmutzung anzeigt.

Dem Nutzer kann hier über die Benutzerschnittstelle beispielsweise über ein visuelles oder akustisches Signal angezeigt werden, dass eine Verschmutzung und somit ein Reinigungsbedarf besteht. Gleichzeitig kann gefragt werden, ob der Nutzer jetzt eine Reinigung durchführen möchte, um die Verschmutzung zu beheben oder nicht. Dies kann dann durch eine Nutzereingabe an der Benutzerschnittstelle beantwortet werden.

Durch ein Anfragen nach einen Reinigungsvorgang kann ein Nutzer automatisch darauf hingewiesen werden, dass der Ist-Zustand und der Soll-Zustand des Haushaltgeräts nicht mehr übereinstimmen und eine Reinigung nötig ist. Gleichzeitig kann der Nutzer selbst bestimmen, ob er die Reinigung durchführen möchte oder nicht, sodass der Nutzer hier nicht bevormundet, sondern lediglich dabei unterstützt wird, den Sauberkeitszustand des Haushaltgeräts im Blick zu behalten.

Das Verfahren kann einen Schritt des Einlesens eines Terminierungssignals zum Festlegen eines Termins für einen Reinigungsvorgang über die Benutzerschnittstelle und mit einem erneuten Schritt des Ausgebens des Anfragesignals zu dem Termin über die Benutzerschnittstelle umfassen.

Das Terminierungssignal kann hier den festgelegten Termin für die nächste Reinigung repräsentieren und kann beispielsweise durch einen Algorithmus bereitgestellt werden, oder aber auch durch eine Nutzereingabe an der Benutzerschnittstelle.

Gerade die Möglichkeit für den Nutzer, einen individuellen Termin für die nächste Reinigung festzulegen kann vorteilhaft sein, wenn beispielsweise, wie vorangehend beschrieben, eine Verschmutzung festgestellt wird, der Nutzer jedoch angibt die Reinigung nicht sofort durchführen zu wollen. So kann der Nutzer selbst festlegen, wann er die Reinigung stattdessen durchführen möchte, womit außerhalb dieses festgelegten Termins nicht immer wieder unnötig ein Hinweis ausgegeben werden muss, um den Nutzer an eine Reinigung zu erinnern, was den Nutzer womöglich stören könnte.

Durch das erneute Ausgeben des Anfragesignals zu dem festgelegten Termin wiederum kann der Nutzer an die Notwendigkeit einer Reinigung erinnert werden. Gleichzeitig kann auch hier der Nutzer angeben, ob er die Reinigung durchführen möchte oder nicht und gegebenenfalls einen neuen Termin festlegen.

Der Nutzer kann somit das Verfahren optimal an seine Bedürfnisse anpassen, womit der Komfort und die Zufriedenheit des Nutzers gesteigert werden können.

Von Vorteil ist des Weiteren eine Ausführungsform des vorgestellten Ansatzes als eine Vorrichtung zur Unterstützung eines Nutzers bei einer Reinigung eines Haushaltgeräts, das eine UV-Lichtquelle zum Beleuchten eines Innenraums des Haushaltgeräts und eine Bilderfassungseinrichtung zum Erfassen eines Abbilds des Innenraums aufweist, wobei die Vorrichtung ausgebildet ist, um die Schritte des Verfahrens, wie es vorangehend beschrieben ist, in entsprechenden Einheiten auszuführen und/oder anzusteuern.

Durch eine Ausführungsform der Erfindung als eine solche Vorrichtung kann die der Erfindung zugrunde liegende Aufgabe schnell und effizient gelöst werden.

Der hier vorgestellte Ansatz schafft somit ferner eine Vorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Vorrichtung kann die der Erfindung zugrunde liegende Aufgabe schnell und effizient gelöst werden.

Die Vorrichtung kann ausgebildet sein, um Eingangssignale einzulesen und unter Verwendung der Eingangssignale Ausgangssignale zu bestimmen und bereitzustellen. Ein Eingangssignal kann beispielsweise ein über eine Eingangsschnittstelle der Vorrichtung einlesbares Sensorsignal darstellen. Ein Ausgangssignal kann ein Steuersignal oder ein Datensignal darstellen, das an einer Ausgangsschnittstelle der Vorrichtung bereitgestellt werden kann. Die Vorrichtung kann ausgebildet sein, um die Ausgangssignale unter Verwendung einer in Hardware oder Software umgesetzten Verarbeitungsvorschrift zu bestimmen. Beispielsweise kann die Vorrichtung dazu eine Logikschaltung, einen integrierten Schaltkreis oder ein Softwaremodul umfassen und beispielsweise als ein diskretes Bauelement realisiert sein oder von einem diskreten Bauelement umfasst sein.

Ein Haushaltgerät, insbesondere Kühlgerät, weist vorteilhafterweise eine UV-Lichtquelle zum Beleuchten eines Innenraums des Haushaltgeräts, eine Bilderfassungseinrichtung zum Erfassen eines Abbilds des Innenraums und eine Vorrichtung, wie sie vorangehend beschrieben ist, auf.

Damit können sich bei der Reinigung des Haushaltgeräts die Schritte des Verfahrens, wie es vorangehend beschrieben ist, optimal durchführen lassen, womit sich die Vorteile durch eine Unterstützung bei der Reinigung des Haushaltgeräts durch dieses Verfahren realisieren lassen können.

Von Vorteil ist auch ein Computer-Programmprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann. Wird das Programmprodukt oder Programm auf einem Computer oder einer Steuereinheit ausgeführt, so kann das Programmprodukt oder Programm zur Durchführung, Umsetzung und/oder Ansteuerung der Schritte des Verfahrens nach einer der hier beschriebenen Ausführungsformen verwendet werden.

Auch wenn der beschriebene Ansatz anhand eines Haushaltgeräts beschrieben wird, kann der hier beschriebene Ansatz entsprechend im Zusammenhang mit einem gewerblichen oder professionellen Gerät, beispielsweise einem medizinischen Gerät, wie einem Reinigungs- oder Desinfektionsgerät, einem Kleinsterilisator, einem Großraumdesinfektor oder einer Container-Waschanlage eingesetzt werden.

Ein Ausführungsbeispiel der Erfindung ist in den Zeichnungen rein schematisch dargestellt und wird nachfolgend näher beschrieben. Es zeigt
- Figur 1: eine schematische Darstellung eines Ausführungsbeispiels eines Haushaltgerätes;
- Figur 2: eine schematische Darstellung eines Innenraums eines Ausführungsbeispiels eines Haushaltgerätes mit einer UV-Lichtquelle und einer Bilderfassungseinrichtung;
- Figur 3: eine schematische Darstellung eines Systems aus einem Haushaltgerät mit einer UV-Lichtquelle und einer Bilderfassungseinrichtung, einer Cloud und einem Endgerät;
- Figur 4: ein Ablaufdiagramm eines Ausführungsbeispiels eines Verfahrens Unterstützung eines Nutzers bei einer Reinigung eines Haushaltgeräts;
- Figur 5: ein Ablaufdiagramm eines weiteren Ausführungsbeispiels eines Verfahrens Unterstützung eines Nutzers bei einer Reinigung eines Haushaltgeräts;
- Figur 6: ein Ablaufdiagramm eines Ausführungsbeispiels eines Verfahrens zur Unterstützung eines Nutzers bei einer weiteren Reinigung eines Haushaltgeräts;
- Figur 7: ein Ablaufdiagramm eines weiteren Ausführungsbeispiels eines Verfahrens zur Unterstützung eines Nutzers bei einer weiteren Reinigung eines Haushaltgeräts; und
- Figur 8: ein Ablaufdiagramm eines weiteren Ausführungsbeispiels eines Verfahrens zur Unterstützung eines Nutzers bei einer Reinigung eines Haushaltgeräts.

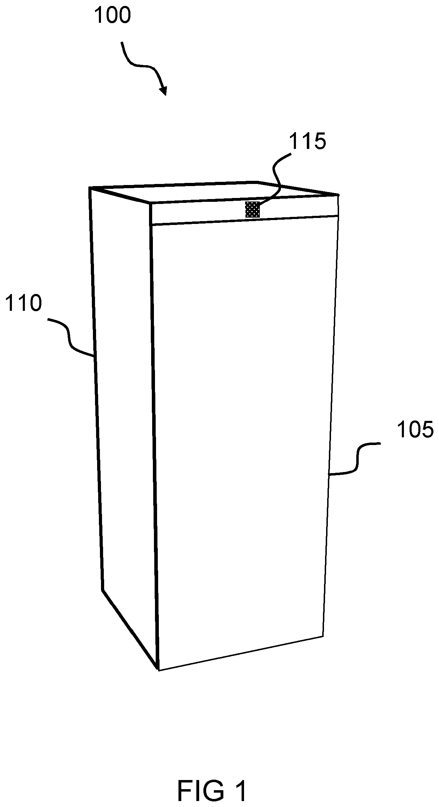

Figur 1 zeigt eine schematische Darstellung eines Ausführungsbeispiels eines Haushaltgerätes 100, welches hier beispielhaft als ein Kühlschrank ausgeformt ist. Der Kühlschrank ist hier geschlossen dargestellt. Dabei verdeckt eine Kühlschranktür 105, synonym als Fronttür bezeichnet, einen Innenraum eines Korpus 110 des Kühlschranks. Der Kühlschrank weist hier außerdem eine Steuervorrichtung 115 auf, welche ausgebildet ist, verschiedene in Figur 1 nicht dargestellte Einrichtungen des Kühlschranks anzusteuern.

Wie nachfolgend näher ausgeführt, kann das Hauhaltgerät 100 einen Nutzer bei einer Reinigung des Haushaltgeräts 100 unterstützten.

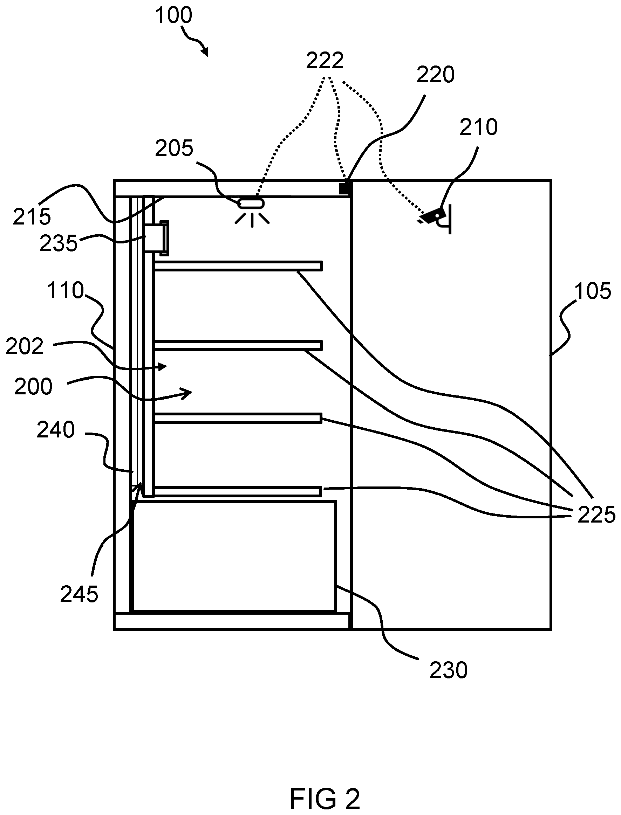

Figur 2 zeigt eine schematische Darstellung eines Innenraums 200 eines Ausführungsbeispiels eines Haushaltgerätes 100 als Kühlschrank, wie er in Figur 1 beschrieben ist.

Die Kühlschranktür 105 ist hier geöffnet, womit der Innenraum 200 des Kühlschranks sichtbar ist. In dem Kühlschrank sind eine Gerätebeleuchtung mit dem Zusatz einer UV-Lichtquelle 205 und eine Bilderfassungseinrichtung 210 angeordnet. Beispielhaft ist die UV-Lichtquelle 205 an einer Decke 215 des Kühlschranks angeordnet, um von oben den gesamten Innenraum 200 des Kühlschranks bis zu einer Rückwand 202 auszuleuchten.

Die Bilderfassungseinrichtung 210, welche im hier dargestellten Ausführungsbeispiel als eine Kamera ausgeformt ist, ist ausgebildet, um den Innenraum 200 des Haushaltgerätes 100 abzubilden. Beispielhaft ist die Bilderfassungseinrichtung 210 an der Kühlschranktür 105 angeordnet und auf den Innenraum 200 ausgerichtet, um ein möglichst übersichtliches Bild des Innenraums 200 erfassen zu können.

Die UV-Lichtquelle 205 und die Kamera 210 sind gemäß einem Ausführungsbeispiel mit einem Kommunikationsmodul 220 gekoppelt, das beispielhaft ein Bestandteil des Haushaltgerätes 100 ist. Das Kommunikationsmodul 220 ist ausgebildet, Bilder von der Kamera 210 zu empfangen und ein Ein- bzw. Ausschalten der UV-Lichtquelle 205 anzusteuern.

Die UV-Lichtquelle 205, die Kamera 210 und das Kommunikationsmodul 220, synonym als Steuerung oder Elektronik bezeichnet, bilden zusammen einen Hygiene-Assistenten 222 aus, welcher einen Nutzer bei einer Reinigung des Kühlschranks 100 unterstützt. Dies wird in den nachfolgenden Figuren näher beschrieben.

Im Innenraum 200 des Kühlschranks sind beispielhaft mehrere Ablageflächen 225 auf verschiedenen Höhen innerhalb des Korpus 110 des Kühlschranks und eine Gemüseschublade 230 an einem Boden des Kühlschranks 100 angeordnet. Zum Kühlen des Innenraums 200 weist der Kühlschrank außerdem einen Lüfter 235, einen Verdampfer 240 und einen Luftkanal 245 auf.

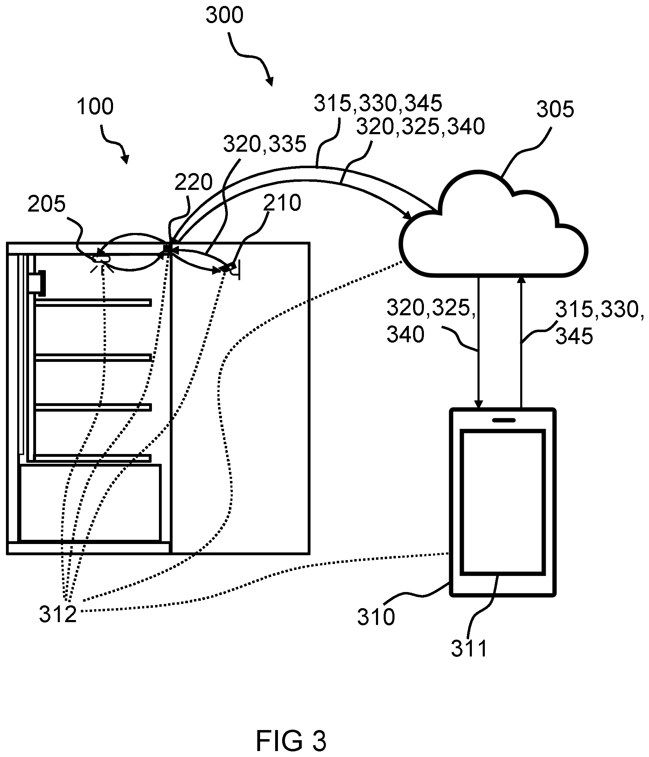

Figur 3 zeigt eine schematische Darstellung eines Systems 300 aus einem Haushaltgerät 100, beispielhaft in Form eines Kühlschranks, mit der UV-Lichtquelle 205, der Bilderfassungseinrichtung in Form der Kamera 210, wie diese anhand der Figuren 1 bis 2 beschrieben sind, einer Cloud 305 und einem Endgerät 310, welches gemäß dem hier dargestellten Ausführungsbeispiel als ein mobiles Gerät, beispielsweise ein Mobiltelefon bzw. Smartphone, ausgeformt ist. Das Endgerät 310 umfasst eine Benutzerschnittstelle 311, hier beispielhaft in Form eines berührungsempfindlichen Bildschirms. Hier bilden die UV-Lichtquelle 205, das Kommunikationsmodul 220, die Kamera 210, die Cloud 305 und das hier beispielhaft als Mobiltelefon ausgeführten Endgeräts 310 als Gesamtsystem zusammen einen Hygiene-Assistenten 312. Durch die Ergänzung der Cloud 305 und des Endgeräts310 im Vergleich zu dem Hygiene-Assistenten 222 aus Figur 2 wird dem Nutzer hier die Möglichkeit geboten, bequem über das eigene Endgerät310 gegebenenfalls die Vorgänge des Hygiene-Assistenten 222 zu steuern. Optional ist das Kommunikationsmodul 220 extern zu dem Haushaltgerät 100 angeordnet, beispielsweise als ein Bestanteil des Endgeräts 310.

Zur Unterstützung eines Nutzers bei einer Reinigung des Haushaltgerätes 100 wird zunächst ein Reinigungsmodus aktiviert, welcher die optimalen Bedingungen für eine Reinigung schafft.

Gemäß dem hier dargestellten Ausführungsbeispiel macht der Nutzer hier eine Eingabe an dem Mobiltelefon 310, woraufhin ein Aktivierungssignal 315 bereitgestellt und an die Cloud 305 übertragen wird, welche das Aktivierungssignal 315 wiederum an das Kommunikationsmodul 220 des Kühlschranks übermittelt, woraufhin dieses das Aktivieren des Reinigungsmodus ansteuert. Der Nutzer kann daraufhin die Reinigung des Haushaltgerätes 100, hier des Kühlschranks, durchführen.

Anschließend wird der Kühlschrank zunächst mit der UV-Lichtquelle 205 ausgeleuchtet, sodass für das menschliche Auge unsichtbare Verschmutzungen sichtbar werden. Die UV-Lichtquelle 205 wird dafür von dem Kommunikationsmodul 220 angesteuert, um diese einzuschalten.

Dann steuert das Kommunikationsmodul 220 ein Erfassen eines zweiten Abbilds 320, synonym als zweites Kamerabild bezeichnet, des Innenraums des Kühlschranks bei eingeschalteter UV-Lichtquelle 205 durch die Kamera 210 an. Das zweite Abbild 320 wird an das Kommunikationsmodul 220 übermittelt, welches ansprechend auf einen Empfang des zweiten Abbilds die UV-Lichtquelle 205 ansteuert, um diese auszuschalten.

Daraufhin wird das zweite Abbild 320 mit einem bereits vorhandenen ersten Abbild 325, synonym als erstes Kamerabild bezeichnet, verglichen, um das Reinigungsergebnis zu beurteilen, wobei das erste Abbild 325 gemäß diesem Ausführungsbeispiel ein gespeichertes Referenzbild ist, welches den Innenraum des Kühlschranks 100 in einem gereinigten Zustand abbildet. Dazu wird das erste Abbild 325 beispielsweise aus einer Speichereinrichtung des Hauhaltgeräts 100, des Endgeräts 310 oder der Cloud 305 ausgelesen.

Bei dem Vergleich gibt es zwei Möglichkeiten, je nachdem ob ein Nutzer selbst den Vergleich unternehmen möchte, oder dies automatisch durch das Kommunikationsmodul 220 geschehen soll:
Möchte ein Nutzer selbst den Vergleich unternehmen, so werden das erste Abbild 325 und das zweite Abbild 320 direkt oder beispielsweise über die Cloud 305 an das Endgerät310 des Nutzers übermittelt. Die Abbilder 320, 325 werden hier über die Benutzerschnittstelle 311 des Endgeräts310 ausgegeben, sodass der Nutzer die Abbilder 320, 325 vergleichen kann, um das Reinigungsergebnis zu beurteilen.

Der Nutzer tätigt daraufhin eine Eingabe an der Benutzerschnittstelle 311 des Endgeräts310, worauf ein Ergebnissignal 330 bereitgestellt wird, welches von dem Endgerät310 direkt oder über die Cloud 305 an das Kommunikationsmodul 220 übermittelt wird. Das Ergebnissignal 330 repräsentiert hierbei ein zufriedenstellendes Reinigungsergebnis oder ein nichtzufriedenstellendes Reinigungsergebnis, je nach Eingabe des Nutzers.

Soll der Vergleich des ersten Abbilds 325 und des zweiten Abbilds 320 automatisch durchgeführt werden, so erfolgt der Vergleich beispielsweise unter Verwendung des Kommunikationsmoduls 220, welches hierbei wiederum selbst das Ergebnissignal 330 bereitstellt. Dazu ist das Kommunikationsmodul 220 ausgebildet, um ein geeignetes Vergleichsverfahren durchzuführen. Sollte das Ergebnissignal 330 in diesem Fall das nichtzufriedenstellende Reinigungsergebnis anzeigen, so steuert das Kommunikationsmodul 220 beispielsweise über die Cloud 305 und das Endgerät310 eine Ausgabe eines Hinweises für den Nutzer an, sodass dem Nutzer angezeigt wird, dass das Reinigungsergebnis nicht optimal ist. Der Hinweis wird beispielsweise über die Benutzerschnittstelle 311 angezeigt.

In diesem Fall kann der Nutzer den Reinigungsvorgang so lange wiederholen, bis ein zufriedenstellendes Reinigungsergebnis erreicht wird.

Sobald dieses zufriedenstellende Reinigungsergebnis vorliegt, wird das zuletzt erfasste zweite Abbild 320 gemäß einem Ausführungsbeispiel als ein aktuelles erstes Abbild, also ein neues Referenzbild, gespeichert, sodass es bei einem weiteren Reinigungsvorgang das erste Abbild 325 ersetzt. Optional wird das Datum des erfolgreich abgeschlossenen Reinigungsvorgangs ebenfalls abgespeichert und der Reinigungsmodus wieder deaktiviert.

Nach einer vorbestimmten Zeitspanne, welche der Nutzer selbst bestimmen kann, wird gemäß einem Ausführungsbeispiel ein Sauberkeitszustand des Kühlschranks 100 ermittelt. Dazu wird von der Kamera 210 ein aktuelles zweites Abbild 335 des Innenraums des Kühlschranks 100 erfasst und an das Kommunikationsmodul 220 bereitgestellt.

Hier erfolgt dann ein Vergleich zwischen dem aktuellen zweiten Abbild 335 und dem gespeicherten neuen Referenzbild, also dem abgespeicherten letzten zweiten Abbild 320 des vorigen Reinigungsvorgangs. Es wird also der aktuelle Zustand des Innenraums des Kühlschranks 100 mit dem Zustand nach einer erfolgreichen Reinigung verglichen.

Anschließend wird ein Zustandssignal bereitgestellt, welches je nach Ergebnis eine Sauberkeit oder eine Verschmutzung des Innenraums anzeigt. In dem Fall, dass das Zustandssignal die Verschmutzung anzeigt, wird anschließend ein Anfragesignal 340 bereitgestellt, welches über die Cloud 305 an das Endgerät310 übermittelt wird, wo es ausgegeben wird, um bei dem Nutzer anzufragen, ob er jetzt eine Reinigung durchführen möchte.

Der Nutzer kann entsprechend eine Eingabe machen, durch welche entweder das Aktivierungssignal 315 bereitgestellt wird, wenn der Nutzer jetzt den Reinigungsvorgang starten möchte, oder ein Terminierungssignal 345 bereitgestellt wird, durch welches ein neuer Termin für die nächste Reinigung festgelegt wird. Der Nutzer wird hierbei über eine Ausgabe an dem Endgerät310 gefragt, ob er einen neuen Termin festlegen möchte. Möchte der Nutzer keinen Termin festlegen, so wird automatisch ein neuer Termin festgelegt, beispielsweise nach einem erneuten Ablauf der vorbestimmten Zeitspanne.

Möchte der Nutzer wiederum einen individuellen Termin festlegen, so kann er dies ebenfalls über die Eingabe an dem Endgerät310 tätigen. Das Terminierungssignal 345 wird dann von dem Endgerät310 an die Cloud 305 und von dort an das Kommunikationsmodul 220 übertragen.

Der Kühlschrank braucht also gemäß dem hier dargestellten Ausführungsbeispiel keine eigene Benutzerschnittstelle. Stattdessen kann diese Rolle von dem Endgerät310 des Nutzers erfüllt werden, welches direkt oder über die Cloud 305 mit dem Kommunikationsmodul 220 des Kühlschranks kommunizieren kann.

Gemäß einem alternativen Ausführungsbeispiel wird anstelle des Endgeräts 310 eine Benutzerschnittstelle des Haushaltgeräts 100 verwendet. In diesem Fall ist eine Kopplung des Haushaltgeräts 100 mit dem Endgerät 310 oder der Cloud 305 nicht erforderlich.

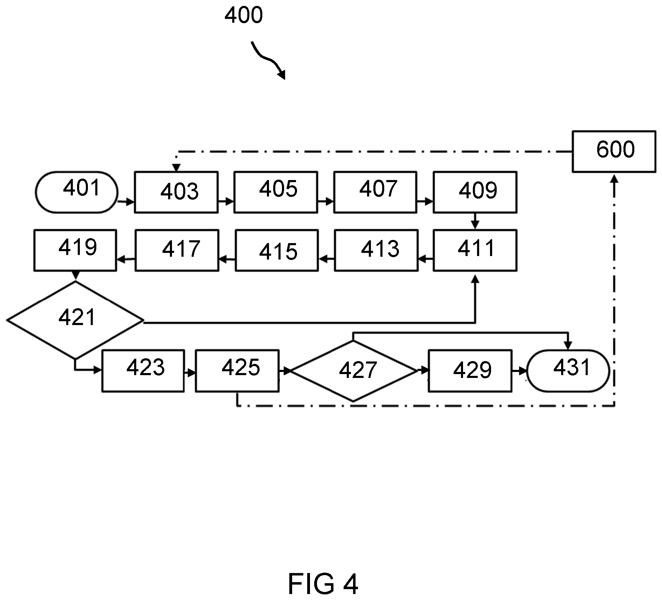

Figur 4 zeigt ein Ablaufdiagramm eines Ausführungsbeispiels eines Verfahrens 400 Unterstützung eines Nutzers bei einer Reinigung eines Haushaltgeräts. Das Verfahren 400 wird hier anhand des Ausführungsbeispiels des Haushaltgeräts als Kühlschrank als Teil eines Systems 300, wie es anhand von Figur 3 beschrieben ist, veranschaulicht.

In einem ersten Schritt 401 wird ein Reinigungsvorgang gestartet. Als Vorbereitung für das Reinigen wird daraufhin in einem Schritt 403 ein Reinigungsmodus des Kühlschranks aktiviert, um gute Bedingungen für einen Nutzer zur Reinigung zu schaffen.

In einem anschließenden Schritt 405 wird die UV-Lichtquelle des Kühlschranks aktiviert, damit in einem folgenden Schritt 407 ein erstes Abbild von der Kamera erfasst werden kann, um den aktuellen Sauberkeitszustand des Kühlschranks 100 abzubilden.

In einem Schritt 409 wird anschließend die UV-Lichtquelle wieder ausgeschaltet, sodass in einem Schritt 411 die Reinigung durchgeführt werden kann. Sobald die Reinigung abgeschlossen ist, wird in einem Schritt 413 die UV-Lichtquelle wieder eingeschaltet, um bei der UV-Beleuchtung in einem Schritt 415 ein zweites Abbild mittels der Kamera zu erfassen, welches den Sauberkeitszustand des Kühlschranks 100 nach der Reinigung abbildet. Anschließend wird die UV-Lichtquelle in einem Schritt 417 wieder ausgeschaltet.

In einem Schritt 419 werden das erste Abbild und das zweite Abbild schließlich an einer Benutzerschnittstelle, wie beispielsweise dem Endgerät310 aus Figur 3, ausgegeben, sodass der Nutzer das Reinigungsergebnis durch einen Vergleich dieser beiden Abbilder beurteilen kann.

Durch eine Eingabe des Nutzers wird daraufhin in einem Schritt 421 ein Ergebnissignal bereitgestellt. War das Reinigungsergebnis nicht zufriedenstellend für den Nutzer, so springt das Verfahren 400 ansprechend auf das Ergebnissignal zurück zum Schritt 411: eine erneute Reinigung wird durchgeführt und die anschließenden Schritte wiederholt, um das neue Ergebnis erneut zu beurteilen.

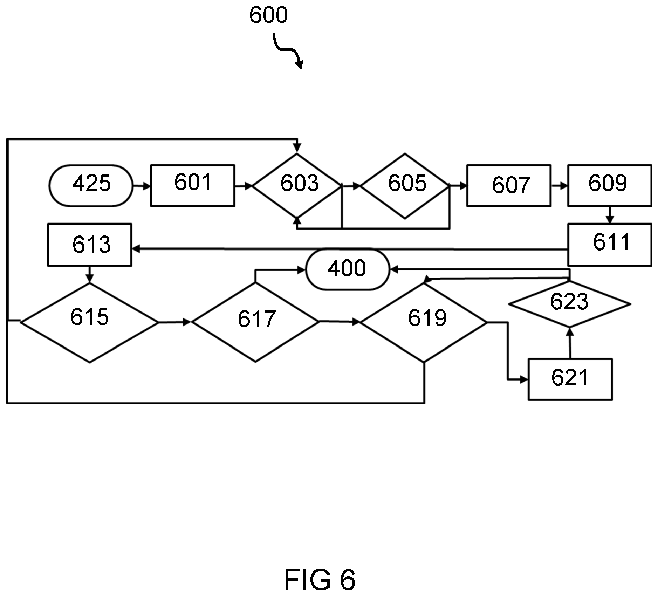

War das Reinigungsergebnis hingegen zufriedenstellend für den Nutzer, so geht das Verfahren 400 ansprechend auf das Ergebnissignal in einen Schritt 423 über, in welchem der Reinigungsmodus ausgeschaltet wird. In einem Schritt 425 wird anschließend das Datum der Reinigung gespeichert. Ansprechend auf das gespeicherte Datum wird nach einer vorbestimmten Zeitspanne ein weiteres Verfahren 600 eingeleitet, welches in Figur 6 beschrieben wird.

Optional weist das Verfahren 400 gemäß dem hier dargestellten Ausführungsbeispiel außerdem einen Schritt 427 auf, in welchem über die Benutzerschnittstelle, also beispielsweise das Endgerät310 aus Figur 3, eine Anfrage an den Nutzer ausgegeben wird, ob das letzte zweite Abbild als ein Referenzbild hinterlegt werden soll, um bei späteren Reinigungsvorgängen als Vorbild für ein zufriedenstellendes Reinigungsergebnis zu dienen.

Bestätigt der Nutzer über eine Eingabe, dass er das zweite Abbild als Referenzbild hinterlegen möchte, so wird das zweite Abbild in einem Schritt 429 als Referenzbild gespeichert. Anschließend wird in einem Schritt 431 das Verfahren 400 beendet.

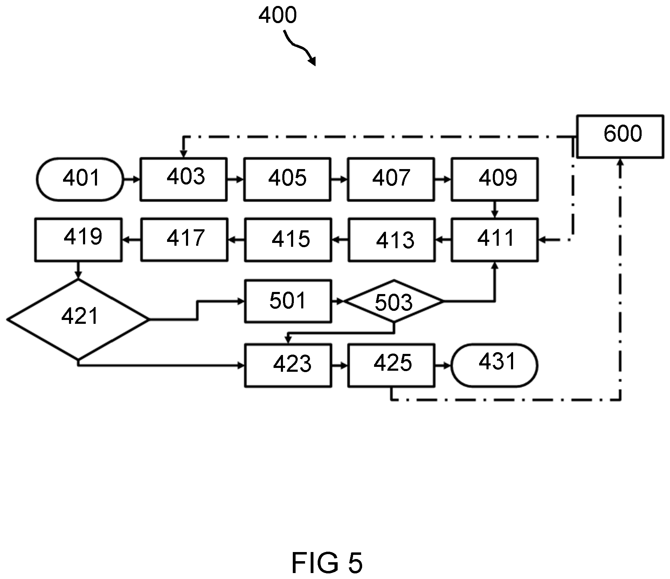

Figur 5 zeigt ein Ablaufdiagramm eines weiteren Ausführungsbeispiels des Verfahrens 400, wie es anhand von Figur 4 beschrieben ist, zur Unterstützung eines Nutzers bei einer Reinigung eines Haushaltgeräts, wobei das Verfahren 400 hier ebenfalls anhand des Ausführungsbeispiels des Haushaltgeräts als Kühlschrank als Teil des Systems 300, wie es anhand von Figur 3 beschrieben ist, veranschaulicht wird.

Die Schritte 401 bis 417 sind dabei die gleichen wie in dem Ausführungsbeispiel des Verfahrens 400 aus Figur 4. Im Schritt 419 werden hier jedoch nicht das erste Abbild und das zweite Abbild an der Benutzerschnittstelle für den Nutzer ausgegeben. Stattdessen erfolgt hier ein Abgleich zwischen dem ersten Abbild und dem zweiten Abbild automatisch durch die Steuerung des Kühlschranks 100 selbst, beispielsweise durch eine KI.

Im Schritt 421 wird hier wieder das Ergebnissignal bereitgestellt, jedoch nicht durch eine Eingabe durch den Nutzer. Das Ergebnis wird hier stattdessen direkt an der Steuerung selbst bereitgestellt.

Wenn das Ergebnissignal anzeigt, dass das Reinigungsergebnis zufriedenstellend ist, geht das Verfahren 400 hier direkt in den Schritt 423 über, in welchem der Reinigungsmodus ausgeschaltet wird. Darauf folgt das Speichern des Reinigungsdatums im Schritt 425, woraufhin das Verfahren 400 im Schritt 431 beendet wird. Auch gemäß dem hier dargestellten Ausführungsbeispiel wird ansprechend auf das gespeicherte Datum nach einer vorbestimmten Zeitspanne ein weiteres Verfahren 600 eingeleitet, welches in Figur 6 beschrieben wird.

Wenn das Ergebnissignal aus Schritt 421 jedoch anzeigt, dass das Reinigungsergebnis nicht zufriedenstellend ist, wird in einem Schritt 501 ein Hinweis an der Benutzerschnittstelle für den Nutzer ausgegeben. Dem Nutzer wird mittels des Hinweises mitgeteilt, wo noch Verschmutzungen vorhanden sind und wo dementsprechend noch intensiver gereinigt werden müsste, um den gewünschten Zustand und ein zufriedenstellendes Reinigungsergebnis zu erhalten.

In einem Schritt 503 wird anschließend eine Anfrage an der Benutzerschnittstelle ausgegeben, ob der Nutzer nun eine erneute Reinigung durchführen will, um das zufriedenstellende Reinigungsergebnis zu erzielen. Möchte der Nutzer dies tun, so springt das Verfahren 400 zurück zum Schritt 411, um eine erneute Reinigung durchzuführen. Möchte der Nutzer jetzt keine weitere Reinigung vornehmen, so geht das Verfahren 400 wiederum in den Schritt 423 über, um den Reinigungsmodus zu beenden.

Figur 6 zeigt ein Ablaufdiagramm eines Ausführungsbeispiels eines Verfahrens 600 zur Unterstützung eines Nutzers bei einer weiteren Reinigung eines Haushaltgeräts. Das Verfahren 600 wird hier anhand des Ausführungsbeispiels des Haushaltgeräts als Kühlschrank als Teil eines Systems 300, wie es anhand von Figur 3 beschrieben ist, veranschaulicht.

Das Verfahren geht dabei von dem Schritt 425 aus dem Verfahren 400, wie es anhand der Figuren 4 oder 5 beschrieben ist, aus, in welchem nach einem ersten Reinigungsvorgang das Datum dieses ersten Reinigungsvorgangs abgespeichert wurde.

Gemäß einem Ausführungsbeispiel umfasst das Verfahren 600 einen Schritt 601, in welchem eine Zeitspanne festgelegt wird, nach welcher periodisch ein Sauberkeitszustand des Kühlschranks 100 ermittelt wird, beispielsweise alle zwei Tage nach der ersten Reinigung. Das Festlegen erfolgt gemäß einem Ausführungsbeispiel durch eine Eingabe des Nutzers an der Benutzerschnittstelle.

In einem weiteren Schritt 603 wird überprüft, ob die in Schritt 601 festgelegte Zeitspanne vergangen ist. Ist dies nicht der Fall, so wird der Schritt 603 so lange wiederholt, bis die festgelegte Zeitspanne vergangen ist.

Sobald dies der Fall ist, geht das Verfahren 600 in einen Schritt 605 über, in welchem überprüft wird, ob die festgelegte Zeitspanne seit der letzten Ermittlung des Sauberkeitszustands des Kühlschranks vergangen ist. Ist dies nicht der Fall, geht das Verfahren 600 wieder in den Schritt 603 über.

Wird in Schritt 605 hingegen festgestellt, dass auch seit der letzten Ermittlung des Sauberkeitszustands des Kühlschranks die festgelegte Zeitspanne vergangen ist, so erfolgt anschließend ein Schritt 607, in welchem erneut die UV-Lichtquelle eingeschaltet wird. In einem nächsten Schritt 609 wird ein aktuelles zweites Abbild des Innenraums des Kühlschranks erfasst, welches den Kühlschrank im aktuellen Zustand abbildet, um anhand des aktuellen zweiten Abbilds den Sauberkeitszustand des Kühlschranks zu beurteilen. In einem weiteren Schritt 611 wird die UV-Lichtquelle anschließend wieder ausgeschaltet.

Darauf folgt ein Schritt 613, in welchem das in Schritt 609 erfasste aktuelle zweite Abbild mit einem ersten Abbild, also einem Referenzbild verglichen wird, welches den Kühlschrank im gereinigten Zustand abbildet. Gemäß einem Ausführungsbeispiel handelt es sich bei diesem Referenzbild um das im Verfahren 400 aus den Figuren 4 oder 5 im Schritt 429 gespeicherte zweite Abbild des Kühlschranks nach der erfolgreich durchgeführten Reinigung.

Gemäß einem Ausführungsbeispiel werden im Schritt 613 das Referenzbild und das in Schritt 609 erfasste aktuelle zweite Abbild an der Benutzerschnittstelle des Kühlschranks ausgegeben, sodass der Nutzer selbst beurteilen kann, ob der Kühlschrank verschmutzt ist und somit eine weitere Reinigung nötig ist, oder ob der Kühlschrank noch sauber ist.

In einem Schritt 615 erfolgt dann ein Einlesen eines Ergebnissignals, welches den beurteilten Sauberkeitsstatus des Kühlschranks repräsentiert. Gemäß einem Ausführungsbeispiel wird das Ergebnissignal über eine Eingabe durch den Nutzer bereitgestellt.

Wenn das eingelesene Ergebnissignal anzeigt, dass der Kühlschrank noch angemessen sauber ist, dann springt das Verfahren 600 zurück zum Schritt 603.

Wenn das eingelesene Ergebnissignal hingegen anzeigt, dass der Kühlschrank schmutzig ist und somit eine weitere Reinigung nötig ist, wird in einem Schritt 617 eine Anfrage an der Benutzerschnittstelle des Kühlschranks ausgegeben, ob der Nutzer jetzt eine weitere Reinigung durchführen möchte. Wenn der Nutzer festlegt, dass er jetzt eine weitere Reinigung durchführen möchte, beispielsweise durch eine Eingabe an der Benutzerschnittstelle, geht das Verfahren 600 hier wieder in das Verfahren 400, wie es anhand der Figuren 4 oder 5 beschrieben ist, über, um die weitere Reinigung durchzuführen.

Gibt der Nutzer hingegen an, dass er zum jetzigen Zeitpunkt keine weitere Reinigung des Kühlschranks unternehmen möchte, erfolgt in einem Schritt 619 ein weiteres Ausgeben einer Anfrage an der Benutzerschnittstelle, ob der Nutzer einen individuellen Termin für eine weitere Reinigung festlegen möchte. Wenn der Nutzer hier festlegt, dass er keinen individuellen Termin für die weitere Reinigung festlegen möchte, springt das Verfahren 600 an dieser Stelle zurück zum Schritt 603.

Wenn der Nutzer wiederum festlegt, dass er einen individuellen Termin für die weitere Reinigung des Kühlschranks festlegen möchte, erfolgt in einem Schritt 621 ein Einlesen eines Terminierungssignals, welches den Termin für die weitere Reinigung repräsentiert. Gemäß einem Ausführungsbeispiel wird das Terminierungssignal durch eine Eingabe des Nutzers an der Benutzerschnittstelle bereitgestellt.

In einem Schritt 623 wird an dem festgelegten Termin eine Anfrage an der Benutzerschnittstelle ausgegeben, ob der Nutzer den Kühlschrank jetzt reinigen möchte. Gibt der Nutzer an, dass er den Kühlschrank jetzt reinigen möchte, geht das Verfahren 600 an dieser Stelle in das Verfahren 400, wie es anhand der Figuren 4 oder 5 beschrieben ist, über, um die weitere Reinigung durchzuführen.

Gibt der Nutzer im Schritt 623 wiederum an, dass er den Kühlschrank jetzt keine weitere Reinigung des Kühlschranks durchführen möchte, springt das Verfahren 600 zurück zum Schritt 619, um abzufragen, ob der Nutzer einen neuen individuellen Termin festlegen möchte.

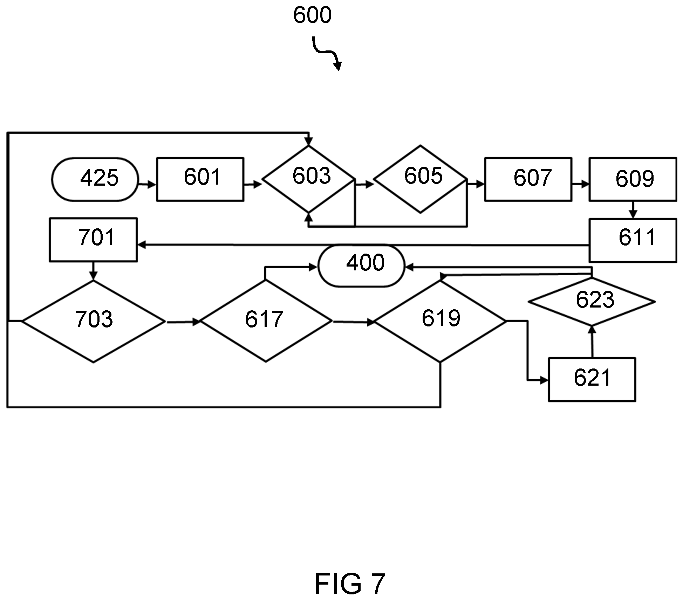

Figur 7 zeigt ein Ablaufdiagramm eines weiteren Ausführungsbeispiels eines Verfahrens 600 zur Unterstützung eines Nutzers bei einer Reinigung eines Haushaltgeräts. Das Verfahren 600 wird hier anhand des Ausführungsbeispiels des Haushaltgeräts als Kühlschrank als Teil eines Systems 300, wie es anhand von Figur 3 beschrieben ist, veranschaulicht.

Die Schritte 601 bis 611 sind dieselben wie in dem Ausführungsbeispiel des Verfahrens 600 aus Figur 6, wobei auch hier das Verfahren 600 aus dem anhand der Figuren 4 bis 5 beschriebenen Verfahren 400 hervorgeht und auf den Schritt 425 folgt, in welchem das Datum der im Verfahren 400 durchgeführten Reinigung gespeichert wurde.

Auf den Schritt 611 folgt hier ein Schritt 701, in welchem automatisch ein Vergleich zwischen einem Referenzbild des Kühlschranks in gereinigtem Zustand, wie beispielsweise das in Schritt 429 des in den Figuren 4 bis 5 beschriebenen Verfahrens 400 abgespeicherte zweite Abbild des Kühlschranks nach dem Reinigungsvorgang des Verfahrens 400, und dem in Schritt 609 erfassten aktuellen zweiten Abbild des Kühlschranks im aktuellen Zustand durchgeführt wird. Im Gegensatz zum Schritt 613 aus dem Ausführungsbeispiel in Figur 6 wird hier keine Ausgabe der Bilder über die Benutzerschnittstelle angesteuert, sondern der Vergleich ist automatisiert, beispielsweise mittels einer Kl.

Anschließend folgt ein Schritt 703, in welchem anhand des Vergleichs in Schritt 701 der aktuelle Sauberkeitsstatus des Kühlschranks ermittelt wird und ein Ergebnissignal bereitgestellt wird, welches den ermittelten Sauberkeitsstatus repräsentiert.

Zeigt das Ergebnissignal einen noch ausreichend sauberen Kühlschrank an, springt auch hier das Verfahren 600 zurück zu dem Schritt 603. Zeigt das Ergebnissignal wiederum an, dass der Kühlschrank nicht mehr ausreichend sauber und dementsprechend eine weitere Reinigung nötig ist, geht das Verfahren 600 hier in den Schritt 617 über, wie er auch schon anhand von Figur 6 beschrieben ist.

Die Schritte 617 bis 623 sind ebenfalls dieselben Schritte wie in Figur 6: Über eine Anfrage an der Benutzerschnittstelle wird der Nutzer gefragt, ob er das Gerät jetzt reinigen möchte, was der Nutzer über eine Eingabe an der Benutzerschnittstelle angeben kann. Gibt der Nutzer an, den Kühlschrank jetzt reinigen zu wollen, geht das Verfahren 600 in das Verfahren 400 über. Möchte der Nutzer den Kühlschrank jetzt nicht reinigen, erfolgt eine erneute Anfrage an der Benutzerschnittstelle, ob der Nutzer einen individuellen Termin für die weitere Reinigung des Kühlschranks festlegen möchte. Der Nutzer kann auch hier eine Eingabe tätigen. Legt der Nutzer fest, keinen Termin festlegen zu wollen, springt auch hier das Verfahren 600 zurück zum Schritt 603. Legt der Nutzer wiederum fest, einen Termin vergeben zu wollen, erfolgt die Festlegung des Termins, auch beispielsweise über eine Eingabe an der Benutzerschnittstelle. Zum festgelegten Termin erfolgt dann eine weitere Anfrage, ob der Nutzer den Kühlschrank jetzt reinigen möchte. Möchte er dies tun, geht an dieser Stelle das Verfahren 600 in das Verfahren 400 über. Möchte er nicht jetzt eine Reinigung durchführen, springt das Verfahren 600 zurück zum Schritt 619.

Im Folgenden wird die Erfindung noch einmal anhand der Figuren 2 bis 7 erläutert.

Der Hygiene Assistent 312 aus Figur 3 ist ein System, das aus verschiedenen Komponenten besteht. Im beispielhaft als Kühlschrank ausgeführten Haushaltgerät 100 ist die UV-Lichtquelle 205 sowie die Kamera 210 implementiert, die mit dem Kommunikationsmodul 220 Daten austauschen kann. Das Kommunikationsmodul 220 tauscht wiederum Daten mit der Cloud 305 aus, welche die Verbindung zu dem Endgerät 310, beispielsweise wie in Figur 3 als Mobiltelefon ausgeformt, herstellt.

Die Aufgabe des Hygiene-Assistenten 312 ist nun, den Nutzer bei der Reinigung seines Haushaltgeräts 100 zu unterstützen. Das UV-Licht der UV-Lichtquelle 205 wird dabei genutzt, um Verschmutzungen im Haushaltgerät 100 sichtbarer zu machen. Die Kamera 210 unterstützt dies durch die Aufnahme der Abbilder 320, 325. Gemäß einem Ausführungsbeispiel werden diese Abbilder 320, 325 weiter mittels Bildverarbeitung genutzt, um die Reinigung zu optimieren.

Gemäß einem Ausführungsbeispiel erfolgt eine Evaluation des Reinigungsergebnisses manuell durch den Nutzer selbst. Ein aufgenommenes Bild unter UV-Licht identifiziert dabei Verschmutzungen, die gegebenenfalls für das menschliche Auge nicht sichtbar sind. Reinigt der Nutzer nun sein Haushaltgerät 100, kann der Hygiene-Assistent 312 das Ergebnis der Reinigung sichtbar machen. Durch das erste Abbild 325 vor der Reinigung und das zweite Abbild 320 nach der Reinigung kann der Nutzer selbst bewerten, wie sauber das Haushaltgerät 100 nach der Reinigung geworden ist. Dies erfolgt gemäß einem Ausführungsbeispiel manuell durch den Nutzer. Ist die Reinigung ausreichend, wird gemäß einem Ausführungsbeispiel das Datum der Reinigung erfasst. Weiterhin wird das zweite Abbild 320 des gereinigten Haushaltgeräts gemäß einem Ausführungsbeispiel als vorbestimmtes Referenzbild für einen automatischen Bildabgleich hinterlegt.

Ist ein Referenzbild des sauberen Kühlschranks 100 hinterlegt, kann die Auswertung im weiteren Verlauf automatisiert durch einen Bildabgleich erfolgen. Der Nutzer kann, muss aber nicht, die Auswertung seiner nächsten Reinigung automatisiert erstellen lassen. Diese automatische Evaluation des Reinigungsergebnisses ist in Figur 5 ausgeführt.

Weiterhin schaltet der Hygiene Assistent gemäß einem Ausführungsbeispiel in einem fest hinterlegtem Zeitintervall eigenständig die UV-Lichtquelle 205 ein und generiert die Abbilder 320, 325 und gibt daraus dem Nutzer Hinweise, welchen Sauberkeitszustand das Haushaltgerät 100 aktuell hat.

Auch dies erfolgt gemäß einem Ausführungsbeispiel wie in Figur 6 durch manuelle Evaluation des Nutzers oder aber auch gemäß einem Ausführungsbeispiel wie in Figur 7 automatisiert mit einem vorbestimmten Referenzbild.

Weiterhin hat der Nutzer gemäß einem Ausführungsbeispiel wie in Figur 6 oder Figur 7 die Möglichkeit, die Reinigung des Haushaltgeräts 100 zu terminieren. Der Prozess sieht wie folgt aus:
Es wird manuell, wie in Figur 6, oder automatisch, wie in Figur 7, ermittelt, dass das Haushaltgerät gereinigt werden muss. Dies kann nicht immer sofort erfolgen. Durch die Funktion der Terminierung kann der Nutzer gemäß einem Ausführungsbeispiel Erinnerungen erhalten, dass die Reinigung ansteht.

Weiterhin kann gemäß einem weiteren Ausführungsbeispiel eine Teilautomatisierung erfolgen, indem beispielsweise der Reinigungsmodus automatisiert eingeschaltet wird.

Eine Ermittlung des Sauberkeitsstatus ist manuell möglich, wie anhand von Figur 6 beschrieben, oder automatisiert möglich, wie anhand von Figur 7 beschrieben. Die anhand der Figuren 4 bis 7 beschriebenen Verfahren können sich gegenseitig unterstützen und bilden zusammen mit der Hardware, beispielsweise bestehend aus UV-Lichtquelle und Kamera ein Ausführungsbeispiel eines Hygiene Assistenten.

Ein großer Vorteil ist, dass der Nutzer nicht bevormundet wird. Das System ist abhängig vom Nutzerverhalten und soll dieses explizit mit einbinden und berücksichtigen. Der Nutzer hat nach wie vor die Hoheit über die Reinigung und den Zustand des Haushaltgeräts.

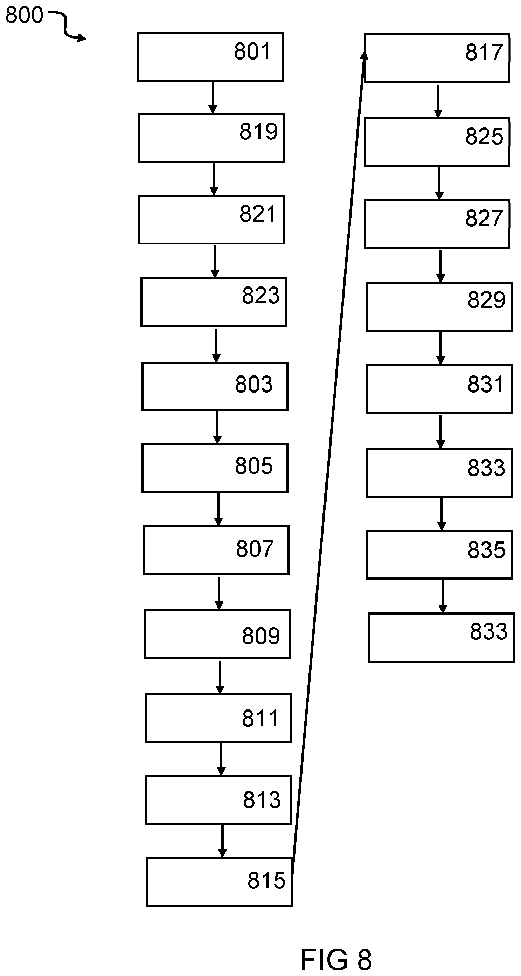

Figur 8 zeigt ein Ablaufdiagramm eines Ausführungsbeispiels eines Verfahrens 800 zur Unterstützung eines Nutzers bei einer Reinigung eines Haushaltgeräts, das eine UV-Lichtquelle zum Beleuchten eines Innenraums des Haushaltgeräts und eine Bilderfassungseinrichtung zum Erfassen eines Abbilds des Innenraums aufweist. Das Verfahren 800 kann als ganzes ausgeführt werden oder es können nur Abschnitte des Verfahrens 800 ausgeführt werden, wie es beispielsweise anhand der vorangegangenen Figuren beschrieben ist.

In einem ersten Schritt 801 des Verfahrens 800 wird ein Reinigungsmodus des Haushaltgeräts aktiviert, welcher die optimalen Bedingungen für einen Nutzer zur Reinigung schafft.

In einem Schritt 803 wird dann die UV-Lichtquelle des Haushaltgeräts eingeschaltet, um in einem Schritt 805 bei UV-Beleuchtung ein zweites Abbild des Innenraums des Haushaltgeräts zu erfassen. In einem Schritt 807 wird anschließend die UV-Lichtquelle wieder ausgeschaltet.

Das in Schritt 805 erfasste zweite Abbild wird dann in einem Schritt 809 zusammen mit einem ersten Abbild für einen Vergleich bereitgestellt. Gemäß einem Ausführungsbeispiel ist das erste Abbild ein vorbestimmtes Referenzbild des Innenraums des Kühlschranks und wird im Schritt 809 aus einer Speichereinheit ausgelesen.

Gemäß einem Ausführungsbeispiel wird der Vergleich des ersten Abbilds und des zweiten Abbilds im Schritt 809 durchgeführt und abhängig von dem Ergebnis des Vergleichs wird dann ein Ergebnissignal bereitgestellt, welches anzeigt, ob der Reinigungsvorgang zu einem zufriedenstellenden oder einem nichtzufriedenstellenden Reinigungsergebnis geführt hat.

Das Verfahren 800 weist dann gemäß einem Ausführungsbeispiel außerdem einen Schritt 811 auf, in welchem über die Benutzerschnittstelle ein Hinweis ausgegeben wird, wenn das in Schritt 809 bereitgestellte Ergebnissignal das nichtzufriedenstellende Reinigungsergebnis anzeigt.

Gemäß einem weiteren Ausführungsbeispiel werden das erste Abbild und das zweite Abbild im Schritt 809 an die Benutzerschnittstelle des Haushaltgeräts zur Anzeige für den Nutzer bereitgestellt. Hier wird dann das Ergebnissignal über die Benutzerschnittstelle bereitgestellt.

Das Verfahren 800 weist hier gemäß einem Ausführungsbeispiel außerdem einen Schritt 813 auf, in welchem das im Schritt 805 erfasste zweite Abbild als ein vorbestimmtes Referenzbild abgespeichert wird, wenn das Ergebnissignal das zufriedenstellende Reinigungsergebnis anzeigt.

Des Weiteren umfasst das Verfahren 800 gemäß einem Ausführungsbeispiel einen Schritt 815, in welchem ein Reinigungsdatum der durchgeführten Reinigung vermerkt wird und einen Schritt 817, in welchem der Reinigungsmodus deaktiviert wird, wenn das bereitgestellte Ergebnissignal das zufriedenstellende Reinigungsergebnis anzeigt.

Gemäß einem weiteren Ausführungsbeispiel umfasst das Verfahren 800 vor dem Schritt 803 einen zusätzlichen Schritt 819, in welchem die UV-Lichtquelle eingeschaltet wird, darauffolgend einen Schritt 821, in welchem das erste Abbild unter Verwendung der Bilderfassungseinrichtung erfasst wird, und anschließend einen Schritt 823, in welchem die UV-Lichtquelle wieder ausgeschaltet wird.

Wenn das Verfahren 800 den Schritt 815 aufweist, in welchem das Reinigungsdatum der durchgeführten Reinigung vermerkt wurde, umfasst das Verfahren 800 außerdem gemäß einem Ausführungsbeispiel einen Schritt 825, in welchem die UV-Lichtquelle nach einer bestimmten Zeitspanne nach dem vermerkten Reinigungsdatum eingeschaltet wird. In einem Schritt 827 wird dann ein aktuelles zweites Abbild des Innenraums des Haushaltgeräts erfasst und in einem Schritt 829 wird die UV-Lichtquelle wieder ausgeschaltet.

In einem Schritt 831 werden dann hier ein erstes Abbild und das in Schritt 827 erfasste aktuelle zweite Abbild für einen Vergleich bereitgestellt, um einen aktuellen Sauberkeitszustand des Innenraums festzustellen. Gemäß diesem Ausführungsbeispiel wird im Schritt 809 das erste Abbild und das aktuelle zweite Abbild verglichen und es wird ansprechend auf ein Ergebnis des Vergleichs ein Zustandssignal bereitgestellt, welches eine Sauberkeit oder eine Verschmutzung des Innenraums des Haushaltgeräts anzeigt.

Wenn dieses Zustandssignal eine Verschmutzung anzeigt, umfasst das Verfahren 800 gemäß einem Ausführungsbeispiel einen Schritt 833, in welchem ein Anfragesignal ausgegeben wird, um über die Benutzerschnittstelle anzufragen, ob ein Reinigungsvorgang gestartet werden soll.

Gemäß einem Ausführungsbeispiel umfasst das Verfahren 800 hier außerdem einen Schritt 835, in welchem ein Terminierungssignal zum Festlegen eines Termins für einen Reinigungsvorgang über die Benutzerschnittstelle eingelesen wird. Zu dem hier festgelegten Termin wird zudem der Schritt 833 wiederholt, um zu dem Termin erneut anzufragen, ob ein Reinigungsvorgang gestartet werden soll.

Wie oben bereits ausgeführt, wird in der vorliegenden Erfindungsmeldung die "Komponente Mensch" berücksichtigt, die bei der Reinigung von Haushaltgeräten unterstützt wird. Es geht vor allem darum, die Nutzer nicht zu bevormunden, sondern ein Angebot der Unterstützung bei der Reinigung ihrer Haushaltgeräte anzubieten. Durch einfache Funktionen soll dazu angeregt werden, das jeweilige Haushaltgerät regelmäßig zu säubern, um damit zur eigenen Gesundheit beizutragen.

## Patentansprüche

1. Verfahren (800) zur Unterstützung eines Nutzers bei einer Reinigung eines Haushaltgeräts (100), das eine UV-Lichtquelle (205) zum Beleuchten eines Innenraums (200) des Haushaltgeräts (100) und eine Bilderfassungseinrichtung (210) zum Erfassen eines Abbilds (320, 325) des Innenraums (200) aufweist, wobei das Verfahren (800) die folgenden Schritte umfasst:
Aktivieren (403; 801) eines Reinigungsmodus zum Ermöglichen eines Reinigungsvorgangs des Innenraums (200) ansprechend auf ein über eine Benutzerschnittstelle (311) eingelesenes Aktivierungssignal (315);
Einschalten (413; 803) der UV-Lichtquelle (205) nach einem Abschluss des Reinigungsvorgangs;
Erfassen (415; 805) eines zweiten Abbilds (320) des Innenraums (200) unter Verwendung der Bilderfassungseinrichtung (210) während die UV-Lichtquelle (205) eingeschaltet ist;
Ausschalten (417; 807) der UV-Lichtquelle (205);
Bereitstellen (421; 809) eines ersten Abbilds (325) und des zweiten Abbilds (320) für einen Vergleich zum Feststellen eines Reinigungsergebnisses des Reinigungsvorgangs.

2. Verfahren (800) gemäß Anspruch 1, wobei im Schritt des Bereitstellens (809) das erste Abbild (325) als ein vorbestimmtes Referenzbild aus einer Speichereinrichtung ausgelesen wird.

3. Verfahren (800) gemäß Anspruch 1, mit einem Schritt des Einschaltens (405; 819) der UV-Lichtquelle (205) ansprechend auf das Aktivieren (403; 801) des Reinigungsmodus, einem Schritt des Erfassens (407; 821) des ersten Abbilds (325) des Innenraums (200) unter Verwendung der Bilderfassungseinrichtung (210) während die UV-Lichtquelle (205) eingeschaltet ist, und einem Schritt des Ausschaltens (409; 823) der UV-Lichtquelle (205), um den Reinigungsvorgang zu ermöglichen.

4. Verfahren (800) gemäß einem der vorangegangenen Ansprüche, wobei im Schritt des Bereitstellens (421; 809) der Vergleich des ersten Abbilds (325) und des zweiten Abbilds (320) durchgeführt und abhängig von einem Ergebnis des Vergleichs ein Ergebnissignal (330) bereitgestellt wird, das anzeigt, ob der Reinigungsvorgang zu einem zufriedenstellenden Reinigungsergebnis oder einem nichtzufriedenstellenden Reinigungsergebnis geführt hat.

5. Verfahren (800) gemäß Anspruch 4, mit einem Schritt des Ausgebens (501; 811) eines Hinweises über die Benutzerschnittstelle (311), wenn das Ergebnissignal (330) das nichtzufriedenstellende Reinigungsergebnis anzeigt.

6. Verfahren (800) gemäß einem der Ansprüche 2 bis 3, wobei im Schritt des Bereitstellens (613; 809) das erste Abbild (325) und das zweite Abbild (320) an die Benutzerschnittstelle (311) zur Anzeige für den Nutzer bereitgestellt werden und über die Benutzerschnittstelle (311) ein Ergebnissignal (330) bereitgestellt wird, das anzeigt, ob der Reinigungsvorgang zu einem zufriedenstellenden Reinigungsergebnis oder einem nichtzufriedenstellenden Reinigungsergebnis geführt hat.

7. Verfahren (800) gemäß einem der Ansprüche 4 bis 6, mit einem Schritt des Abspeicherns (429; 813) des zweiten Abbilds (320) als ein vorbestimmtes Referenzbild in eine Speichereinrichtung, wenn das Ergebnissignal (330) das zufriedenstellende Reinigungsergebnis anzeigt.

8. Verfahren (800) gemäß einem der Ansprüche 4 bis 7, mit einem Schritt des Vermerkens (425; 815) eines Reinigungsdatums und einem Schritt des Deaktivierens (423; 817) des Reinigungsmodus, wenn das Ergebnissignal (330) das zufriedenstellende Reinigungsergebnis anzeigt.

9. Verfahren (800) gemäß Anspruch 8, wobei eine vorbestimmte Zeitspanne nach dem Reinigungsdatum ein Schritt des Einschaltens (607; 825) der UV-Lichtquelle (205), ein Schritt des Erfassens (609; 827) eines aktuellen zweiten Abbilds (320), ein Schritt des Ausschaltens (611; 829) der UV-Lichtquelle (205) und ein Schritt des Bereitstellens (831) eines ersten Abbilds (325) und des aktuellen zweiten Abbilds (320) für einen Vergleich zum Feststellen eines aktuellen Sauberkeitszustands des Innenraums (200) ausgeführt werden, wobei im Schritt des Bereitstellens (831) der Vergleich des ersten Abbilds (325) und des aktuellen zweiten Abbilds (320) durchgeführt und abhängig von einem Ergebnis des Vergleichs ein Zustandssignal bereitgestellt wird, das eine Sauberkeit oder Verschmutzung des Innenraums (200) anzeigt.

10. Verfahren (800) gemäß Anspruch 9, mit einem Schritt des Ausgebens (617; 833) eines Anfragesignals (340) zum Anfragen nach einem Reinigungsvorgangs über die Benutzerschnittstelle (311), wenn das Zustandssignal die Verschmutzung anzeigt.

11. Verfahren (800) gemäß Anspruch 10, mit einem Schritt des Einlesens (621; 835) eines Terminierungssignals (345) zum Festlegen eines Termins für einen Reinigungsvorgang über die Benutzerschnittstelle (311) und mit einem erneuten Schritt des Ausgebens (833) des Anfragesignals (340) zu dem Termin über die Benutzerschnittstelle (311).

12. Vorrichtung zur Unterstützung eines Nutzers bei einer Reinigung eines Haushaltgeräts (100), das eine UV-Lichtquelle (205) zum Beleuchten eines Innenraums (200) des Haushaltgeräts (100) und eine Bilderfassungseinrichtung (210) zum Erfassen eines Abbilds (320, 325) des Innenraums (200) aufweist, wobei die Vorrichtung ausgebildet ist, um die Schritte des Verfahrens (800) gemäß einem der vorangegangenen Ansprüche in entsprechenden Einheiten auszuführen und/oder anzusteuern.

13. Haushaltgerät (100), insbesondere Kühlgerät, mit einer UV-Lichtquelle (205) zum Beleuchten eines Innenraums (200) des Haushaltgeräts (100), einer Bilderfassungseinrichtung (210) zum Erfassen eines Abbilds (320, 325) des Innenraums (200) und einer Vorrichtung gemäß Anspruch 12.

14. Computer-Programmprodukt mit Programmcode zur Durchführung des Verfahrens (800) nach einem der Ansprüche 1 bis 11, wenn das Computer-Programmprodukt auf einer Vorrichtung gemäß Anspruch 12 ausgeführt wird.
